# EUROPEAN PATENT SPECIFICATION

(11) **EP 1 496 357 B1**
(45) Date of publication and mention of the grant of the patent: **22.11.2006**
(21) Application number: 04012554.4
(22) Date of filing: 27.05.2004
(51) Int. Cl.: G01N 31/22, G01N 33/28

(54) **Analytical method and device for determining metal concentration in liquid hydrocarbon matrices**
Analytisches Verfahren und Vorrichtung zur Bestimmung der Metallkonzentration in flüssigen Kohlenwasserstoffen
Procède analytique et dispositif pour la détermination de la concentration d'un métal dans les hydrocarbone liquide

(30) Priority: 08.07.2003 US 615075
(43) Date of publication of application: 12.01.2005
(73) Proprietor: Afton Chemical Intangibles LLC, Richmond, Virginia 23219 (US)
(72) Inventor: Franklin, Randall M., Mechanicsville Virgina 23116 (US); Roos, Joseph W., Mechanicsville Virgina 23116 (US)
(74) Representative: Schwabe - Sandmair - Marx

(56) References cited:
- EP-A- 1 251 346
- NL-A- 8 700 600
- US-A- 3 635 679
- US-A- 3 806 319
- US-A- 3 934 976
- US-A- 3 955 927

## Description

### FIELD OF THE INVENTION

The present invention relates to a device and method for detecting metal concentration in various hydrocarbon matrices, such as for example, gasolines and distillate fuels. The device, also referred to as a kit, of this invention is able to detect and quantify levels of metals, such as manganese in the range between 0.5 - 50 milligrams/liter of metal in the hydrocarbonaceous matrices.

### BACKGROUND

Many metals find their way into combustible hydrocarbons, some by accident, and others intentionally. Monitoring the type and amount of these metals can be useful in enhancing performance and/or minimizing detriments in combustion devices.

Manganese in the form of, for example, methyl cyclopentadienyl manganese tricarbonyl ("MMT") is added to combustible materials for several purposes, including use as an anti-knock agent for spark ignited engines. MMT^{®} is a registered trademark of Ethyl Corporation for its methyl cyclopentadienyl manganese tricarbonyl fuel additive. Another use of MMT^{®} is as an additive to distillate fuels as a smoke suppressant by catalyzing particulate carbon burnout both in compression ignition (diesel) engines and stationary burners. MMT^{®} has been used as a fuel additive for many years and has recently received much attention for the significant improvements it can achieve in particulate emission reduction, smoke suppression, particulate trap regeneration, and scavenging of potential catalyst poisons. Fuel suppliers and customers are very interested in the effective treat rates and cost effectiveness of MMT^{®} as a fuel additive, as well as verifying that a fuel has been properly additized.

US 3,955,927 relates to a method for colorimetrically determining the lead content in gasoline by converting the tetraalkyllead in a gasoline to a dialkyllead diiodide comprising reacting the tetraalkyllead with iodine and exposing the resulting reation mixture to ultraviolet radiation of 2800A to below 4000A whereby said dialkyllead diiodide is formed, adding a pyridylazo hydroxyaryl compound in aqueous solution to the gasoline to produce a color in the aqueous phase corresponding to the initial lead concentration.

Further, US 3,806,319 relates to a process for the detection of trace amounts of lead in unleaded gasoline comprising the steps:
(a) mixing a sample of the gasoline to be tested with a solution of a halogen in an alcohol containing up to 8 carbons;
(b) mixing the resultant mixture with dilute aqueous nitric acid;
(c) separating the aqueous phase form the gasoline phase;
(d) agitating said aqueous phase with sodium sulfite to form a precipitate of lead sulfite;
(e) filtering the mixture to remove therefrom the precipitate;
(f) adding to the precipitate a buffer solution having a pH of about 2.8; and
(g) thereafter adding to said precipitate a solution of sodium rhodizonate.

In addition, US 3,934,976 relates to a method for colorimetrically determining the lead content in gasoline comprising (1) mixing a gasoline sample containing tetraalkyllead with iodine in the presence of a tetraorganoammonium halide and exposing the mixture to ultraviolet radiation to convert the tetraalkyllead to dialkyllead diiodide, (2) mixing the resulting reaction mixture with an aqueous solution of an acidic compound selected from the group consisting of nitric acid and ammonium nitrate and separating the resulting aqueous phase from the organic gasoline phase, and (3) combining the aqueous phase with an alkali metal salt of 4-(2-pyridylazo)-resorcinol in an aqueous solution to produce a color corresponding to the initial lead concentration.

A method for detecting and quantifying manganese in aqueous media is commercially available as "REFLECTOQUANT Manganese Test, distributed by EM SCIENCE, a division of EM Industries, Inc., Gibbstown, New Jersey 08027. This strip is commercially optimized for photometrically determining manganese levels in aqueous media in the range of 0.5 - 45 mg/l Mn. As described in the public literature, however, this method cannot perform the same function in organic media.

Thus, a need has arisen for determining easily, quickly and inexpensively the presence and amount of metals, including manganese, in various hydrocarbon matrices, such as but not limited to fuels.

### SUMMARY OF THE EMBODIMENTS

An embodiment presented herein provides a device for determining metal levels in various hydrocarbon matrices, such as for example, gasolines and distillate fuels. In one embodiment, the metal is manganese.

By "hydrocarbon matrix" herein is meant any hydrocarbonaceous material, fluid, composition or mixture, containing organic chemicals. The hydrocarbon matrices herein can include for example but are not limited to fuels such as gasoline and petroleum distillate, diesel fuel, biodiesel fuel, kerosene, crude oil, refined oil, lubricants, including engine oils, transmission fluids, hydraulic oils, aviation fuels, cutting fluids, extracts from the above, distillate bottoms, fuel oil, and other hydrocarbonaceous matrices.

Another embodiment provides a method for determining metal levels in various hydrocarbon matrices, such as for example, gasolines and distillate fuels. In one embodiment, the metal is manganese.

Accordingly, in one example herein is provided a method for detecting the presence and amount of a metallic species in a hydrocarbon matrix, comprising (a) contacting a hydrocarbon matrix containing an organometallic compound with a solid colorimetric detection material comprising a colorimetric sensitizer chemical able to react with the metal of the organometallic compound; (b) causing a reaction between the metal from the organometallic compound and the colorimetric sensitizer chemical to form an organometallic complex, and (c) detecting the presence of the organometallic complex.

In another example is provided a detection system, device or kit useful in the determination of the amount of manganese in an organo manganese-containing hydrocarbon matrix. As an example, the detection system, device or kit for detecting the presence of metal in a hydrocarbon matrix can include a solid colorimetric detection material comprising at least one colorimetric sensitizer chemical able to react with a metal of an organometallic compound in the hydrocarbon matrix.

It is to be understood that both the foregoing general description and the following detailed description are exemplary and explanatory only and are intended to provide further explanation of the present invention, as claimed.

### DETAILED DESCRIPTION OF EMBODIMENTS

In an example of an embodiment provided herein a method for detecting the presence and amount of metal present as an organometallic compound such as organo manganese in a hydrocarbon matrix is provided that comprises 1) immersing or contacting a colorimetric or material in or with the organometallic-containing hydrocarbon matrix, herein the organometallic compound containing certain organic ligands that bind to the metal atoms, 2) subjecting the material to heat, chemical energy or ultra violet (UV) light, if necessary, to liberate the metal from the organic ligands and deposit the freed metal on or in the solid colorimetric detection material or within its structure, 3) if necessary to achieve colorimetric detection by a sensitizer, adding a sufficient amount (for example, a few drops) of dilute (for example 2% to 10%) basic solution, such as NaOH (Reagent No. 1), followed by adding a sufficient amount (such as a few drops) of aqueous dilute acid, such as acetic acid (for example 5% to 15%) (Reagent No. 2). In an embodiment, the two reagents achieve the oxidation of the metal sufficient for the metal to then react with a colorimetric sensitizer in or on the solid detection material to form an organometallic complex that is colorimetrically detected and quantified using for example the "Rqflex" electronic colorimeter detection unit, (also referred to herein as a "photometer"). The metal is in one example Mn (IV) and is, in one embodiment, colorimetrically quantified using a colorimetric photometer, such as the Merck-distributed electronic meter called "Rqflex." When the treated solid detection material is inserted in or exposed to the photometer, the photometer translates the color intensity resulting from the organometallic complex to a concentration reading, which is then electronically displayed on the instrument.

By "colorimetric detection material" herein is meant any solid article such as a strip, tab, pad, membrane, filter, etc., wherein the material is able to, or contains a substance that is able to, colorimetrically detect or facilitate detection of the presence of an organometallic complex or the metal thereof.

By "dilute basic solution" herein is meant an aqueous basic solution of an alkali or alkaline earth metal salt, including for example NaOH, KOH, Ca(OH)₂, and Mg(OH)₂ or precursors, derivatives or mixtures thereof.

By "dilute acid" herein is meant an aqueous acid solution of a mineral acid or organic acid, such as HCl, acetic acid, etc.

In an embodiment herein, the solid colorimetric detection material is a strip, Reagent No.1, Reagent No. 2, and "Rqflex" electronic colorimetric detection unit useful herein are all commercially available as a kit under the name of "REFLECTOQUANT Manganese Test", distributed by EM SCIENCE, a division of EM Industries, Inc., Gibbstown, New Jersey 08027. However, as supplied commercially the "REFLECTOQUANT Manganese Test" kit is optimized for detecting manganese in aqueous media and does not work for liquid hydrocarbon matrices. The current invention provides both a kit and a method to detect manganese in hydrocarbon media by introducing steps 1 and 2 into the procedure as outlined above.

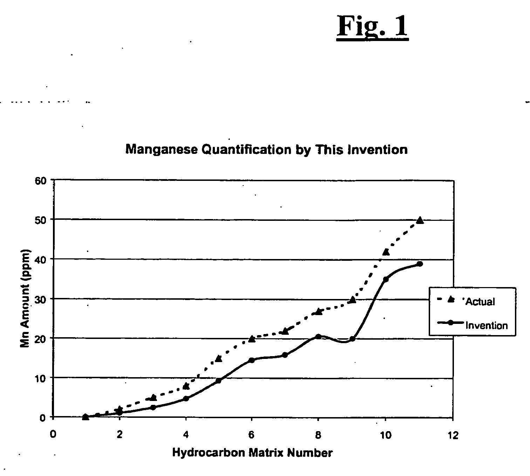

The results validating certain embodiments of this invention are graphically summarized in Figure 1. The data points labeled "Actual" correspond to the amount of manganese pipetted into each fuel (hydrocarbon matrix) as calculated to give the indicated concentrations. Each data point to the curve labeled "Invention" was the average of three determinations carried out by a method of this invention. As can be seen from the relationship between the two data sets, it is possible to generate a calibration curve for this embodiment of the invention method.

The present invention, in an embodiment, converts the organic manganese (such as but not limited to MMT) into an inorganic form (Mn II) and transports it from the organic phase on to an inorganic substrate or matrix on or in a solid colorimetric detection strip or material.

Once adsorbed on the inorganic matrix of the detection strip or dispersed on the aqueous solution, the Mn (II) is oxidized to Mn (IV) by the base/acid reagents for forming the organometallic complex and subsequent detection and quantification as described above.

In contrast to the published aqueous method above, this method of this embodiment involves contacting the solid colorimetric detection material with or immersing it into the hydrocarbon matrix containing the metal such as manganese or iron to layer, adsorb or absorb an organic film on top of or within the inorganic detection material, strip, substrate or matrix. Subjecting this treated detection material to sufficient energy, such as UV radiation, to decompose the organic metallic compound (such as ferrocene and MMT) to inorganic iron or manganese, which adsorbs into or disperses in the inorganic detection material. Treatment with Reagent 1 followed by treatment with by Reagent 2 can oxidize the manganese (II) to manganese (IV) which then binds with the colorimetric indicator chemical in the inorganic detection material to form an organometallic complex. This complex can then be analyzed using a detection device, such as the Rqflex colorimetric electronic meter, to quantify the manganese concentration of the hydrocarbon matrix being analyzed.

The energy applied to decompose the organometallic compound can also be thermal (heat), chemical, sonic, or sunlight. In one example, the wavelength and intensity in joules/mole is designed to match the wavelength of absorption of the organometallic compound.

The method of an embodiment herein can be used to detect and quantify gasoline and diesel fuels treated with the manganese containing additive MMT, for either anti-knock purposes (gasolines) or for particulate emission lowering (distillate fuels). The method can also be used to determine iron, manganese, or other metal concentration in any liquid hydrocarbonaceous matrix. The principles, demonstrated herein, of transporting organic manganese from an organic to an inorganic phase to facilitate detection and quantification by a method developed for aqueous inorganic media can be extended to other metallic elements existing as organometallics in organic media. Examples of such elements in addition to Mn are Cr, Fe, Co, Cu, Zr, Mo, Ru, Rh, Pd, La, Hf, Re, Os, Ir, Pt, Au, Hg, Ce, and other transition metals, lanthanides and actinides that form colored inorganic compounds in the aqueous media. The organo portion of the organometallic compound detectable herein can be any chelate, complex, coordination group, sandwich molecule, solubilizing agent, carrier, salt, crown ether, cryptands, aza-crown ether, spherands, and the like. Thus, for example, the sandwich-like structure of ferrocene and MMT illustrate certain embodiments of the organo portion of the organometallic compound detectable herein.

In another embodiment provided herein is a device, system or kit comprising materials and reagents useful in performing the methods of detection taught herein. Thus another example includes a collection, combination or assembly of some or all of the materials described herein above. Yet another example includes (a) at least one solid colorimetric detection strip with organic ligands able to bind to or bond with certain metal ions to form an organometallic complex and also containing at least one colorimetric indicator chemical, (b) a source or supply of dilute basic solution, such as NaOH, (c) a source or supply of dilute acidic solution, such as acetic acid, (d) optionally an ultraviolet radiation source, and (e) a colorimetric photometer able to detect organometallic complex concentration as a function of color intensity. The UV source useful herein can include, for example, the sun, fluorescent lamps, incandescent lamps, UV irradiation lamps, and other devices able to emit radiation in the wavelength of ultraviolet radiation.

Advantages of this invention include but are not limited to 1) facilitating a rapid spot check of metal additive concentrations in commercial hydrocarbonaceous fuels in the field, 2) simple to operate, requiring little expertise, and 3) it is readily portable.

Other embodiments of the present invention will be apparent to those skilled in the art from consideration of the specification and practice of the invention disclosed herein. It is intended that the specification and examples be considered as exemplary only, with a true scope of the invention being indicated by the following claims.

## Claims

1. A method for detecting the presence of a metallic species in a hydrocarbon matrix, comprising (a) contacting a hydrocarbon matrix containing an organometallic compound with a solid colorimetric detection material comprising a colorimetric sensitizer chemical able to react with the metal of the organometallic compound; (b) causing a reaction between the metal from the organometallic compound and the colorimetric sensitizer chemical to form an organometallic complex, and (c) detecting the presence of the organometallic complex.

2. The method of claim 1, wherein the detection material is exposed to energy to at least partially liberate the metal of the organometallic compound from the compound onto or within the detection material.

3. The method of claim 2, wherein the energy is in the form of heat, is in the form of sonic radiation, is form a chemical reaction due to a chemical added, is in the form of ultraviolet radiation, is in the form of sunlight, or is radiation of a wavelength and intensity (measured in joules/mole) absorbed by the organometallic compound to decompose said compound.

4. The method of claim 1, wherein the hydrocarbon matrix is selected from the group consisting of gasolines, petroleum distillate fuels, distillate fuels, kerosene, diesel fuel, biodiesel fuel, fuel oil, crude oil, refined oil, lubricants, engine oils, transmission fluids, hydraulic oils, aviation fuels, cutting fluids, industrial hydrocarbonaceous waste, and distillate bottoms.

5. The method of claim 1, wherein the hydrocarbon matrix is gasoline, or diesel fuel.

6. The method of claim 1, wherein the metal of the organometallic compound is selected from the group consisting of Mn, Cr, Fe, Co, Cu, Zr, Mo, Ru, Rh, Pd, La, Hf, Re, Os, Ir, Pt, Au, Hg, Ce, lanthanides, and actinides.

7. The method of claim 1, wherein the presence of the organometallic complex is detected by a photometer.

8. The method of claim 1, further comprising the step of combining the detection material from step (b) with an amount of dilute basic solution to the at least partially liberated metal of the organometallic compound.

9. The method of claim 8, further comprising the step of combining the basified metal with a dilute acid to oxidize the metal to an oxidation state able to react with the colorimetric sensitizer chemical.

10. The method of claim 1, wherein the concentration of the organometallic compound in the hydrocarbon matrix is determined by translating the colorimetrically detected color intensity of the organometallic complex into a metal concentration value.

11. The method of claim 1, wherein the metal of the organometallic compound is manganese.

12. The method of claim 10, wherein the organometallic compound is methyl cyclopentadienyl manganese tricarbonyl.

13. A detection system for detecting the presence of metal in a hydrocarbon matrix, said detection comprising a solid colorimetric detection material comprising at least one colorimetric sensitizer chemical able to react with a metal of an organometallic compound in the hydrocarbon matrix.

14. The detection system of claim 13, further comprising a source of radiation to liberate the metal in the organometallic compound from the compound.

15. The detection system of claim 13, further comprising a means for colorimetrically detecting the presence of a metal.

16. The detection system of claim 13, further comprising one or more reagents effective in combining the metal from the organometallic compound with the at least one colorimetric sensitizer chemical.

## Patentansprüche

1. Verfahren zum Nachweis der Gegenwart einer metallischen Spezies in einer Kohlenwasserstoffmatrix, umfassend (a) das In-Kontakt-Bringen einer eine organometallische Verbindung enthaltenden Kohlenwasserstoffmatrix mit einem festen kolorimetrischen Nachweismaterial, umfassend eine kolorimetrische Sensibilisatorchemikalie, die in der Lage ist, mit dem Metall der organometallischen Verbindung zu reagieren, (b) das Veranlassen einer Reaktion zwischen dem Metall aus der organometallischen Verbindung und der kolorimetrischen Sensibilisatorchemikalie, um einen organometallischen Komplex zu bilden, und (c) Nachweisen der Gegenwart des organometallischen Komplexes.

2. Verfahren nach Anspruch 1, bei dem das Nachweismaterial der Einwirkung von Energie ausgesetzt wird, um das Metall der organometallischen Verbindung zumindest teilweise aus der Verbindung auf oder in das Nachweismaterial freizusetzen.

3. Verfahren nach Anspruch 2, bei dem die Energie in Form von Wärme vorliegt, in Form von Schallstrahlung vorliegt, in Form einer chemischen Reaktion aufgrund einer zugesetzten Chemikalie vorliegt, in Form von UV-Licht vorliegt, in Form von Sonnenlicht vorliegt oder eine Strahlung einer Wellenlänge und Intensität (gemessen in Joules/Mol) ist, die von der organometallischen Verbindung absorbiert wird, um diese Verbindung zu zersetzen.

4. Verfahren nach Anspruch 1, bei dem die Kohlenwasserstoffmatrix ausgewählt wird aus der Gruppe, bestehend aus Benzinen, Erdöldestillattreibstoffen, Destillattreibstoffen, Kerosin, Dieseltreibstoff, Biodieseltreibstoff, Treibstofföl, Rohöl, raffiniertem Öl, Schmiermitteln, Motorölen, Getriebefluids, Hydraulikölen, Flugzeugtreibstoffen, Schneidfluids, industriellem kohlenwasserstoffhaltigem Abfall und Destillatbodensätzen.

5. Verfahren nach Anspruch 1, bei dem die Kohlenwasserstoffmatrix Benzin oder Dieseltreibstoff ist.

6. Verfahren nach Anspruch 1, bei dem das Metall der organometallischen Verbindung ausgewählt wird aus der Gruppe, bestehend aus Mn, Cr, Fe, Co, Cu, Zr, Mo, Ru, Rh, Pd, La, Hf, Re, Os, Ir, Pt, Au, Hg, Ce, Lanthanoiden und Actinoiden.

7. Verfahren nach Anspruch 1, bei dem die Gegenwart des organometallischen Komplexes durch ein Photometer nachgewiesen wird.

8. Verfahren nach Anspruch 1, das außerdem den Schritt des Kombinierens des Nachweismaterials aus Schritt (b) mit einer Menge einer verdünnten basischen Lösung zu dem zumindest teilweise freigesetzten Metall der organometallischen Verbindung umfasst.

9. Verfahren nach Anspruch 8, das außerdem den Schritt des Kombinierens des basisch gestellten Metalls mit einer verdünnten Säure zur Oxidation des Metalls zu einem Oxidationszustand umfasst, in dem es mit der kolorimetrischen Sensibilisatorchemikalie reagieren kann.

10. Verfahren nach Anspruch 1, bei dem die Konzentration der organometallischen Verbindung in der Kohlenwasserstoffmatrix **dadurch** bestimmt wird, dass man die kolorimetrisch nachgewiesene Farbintensität des organometallischen Komplexes in einen Wert der Metallkonzentration übersetzt.

11. Verfahren nach Anspruch 1, bei dem das Metall der organometallischen Verbindung Mangan ist.

12. Verfahren nach Anspruch 10, bei dem die organometallische Verbindung Methylcyclopentadienylmangantricarbonyl ist.

13. Nachweissystem zum Nachweis der Gegenwart von Metall in einer Kohlenwasserstoffmatrix, wobei dieser Nachweis festes kolorimetrisches Nachweismaterial umfasst, das mindestens eine kolorimetrische Sensibilisatorchemikalie einschließt, die mit einem Metall einer organometallischen Verbindung in der Kohlenwasserstoffmatrix reagieren kann.

14. Nachweissystem nach Anspruch 13, das außerdem eine Strahlungsquelle umfasst, um das Metall in der organometallischen Verbindung aus der Verbindung freizusetzen.

15. Nachweissystem nach Anspruch 13, das außerdem ein Mittel zum kolorimetrischen Nachweis der Gegenwart eines Metalls umfasst.

16. Nachweissystem nach Anspruch 13, das außerdem ein oder mehrere Reagenzien umfasst, die bewirken, dass das Metall aus der organometallischen Verbindung mit der mindestens einen kolorimetrischen Sensibilisatorchemikalie kombiniert wird.

## Revendications

1. Procédé pour détecter la présence d'une substance métallique dans une matrice d'hydrocarbure, lequel procédé comporte des étapes qui comprenant à :
(a) mettre en contact la matrice d'hydrocarbure contenant un composé organométallique avec un matériau solide de détection colorimétrique qui comprend une substance chimique de sensibilisation colorimétrique capable de réagir avec le métal du composé organométallique,
(b) amener une réaction entre le métal du composé organométallique et la substance chimique de sensibilisation colorimétrique pour former un complexe organométallique et
(c) détecter la présence du complexe organométallique.

2. Procédé selon la revendication 1, dans lequel le matériau de détection est exposé à de l'énergie pour libérer au moins une partie du métal du composé organométallique du composé jusque sur ou dans le matériau de détection.

3. Procédé selon la revendication 2, dans lequel l'énergie présente la forme de chaleur, la forme d'un rayonnement acoustique, la forme d'une réaction chimique suite à l'addition d'une substance chimique, la forme d'un rayonnement ultraviolet, la forme de lumière solaire, un rayonnement dont la longueur d'onde et l'intensité (mesurée en joules/mole) absorbée par le composé organométallique décomposent ledit composé.

4. Procédé selon la revendication 1, dans lequel la matrice d'hydrocarbure est sélectionnée dans l'ensemble constitué des essences, des carburants distillés du pétrole, des carburants distillés, du kérosène, du carburant diesel, du carburant biodiesel, de l'huile combustible, du pétrole brut, du pétrole raffiné, des lubrifiants, des huiles pour moteurs, des fluides de transmission, des huiles hydrauliques, des carburants pour aviation, des fluides de coupe, des déchets industriels d'hydrocarbures et des fonds de colonne de distillation.

5. Procédé selon la revendication 1, dans lequel la matrice d'hydrocarbure est de l'essence ou du carburant diesel.

6. Procédé selon la revendication 1, dans lequel le métal du composé organométallique est sélectionné dans l'ensemble constitué de Mn, Cr, Fe, Co, Cu, Zr, Mo, Ru, Rh, Pd, La, Hf, Re, Os, Ir, Pt, Au, Hg, Ce, les lanthanides et les actinides.

7. Procédé selon la revendication 1, dans lequel la présence du complexe organométallique est détectée par un photomètre.

8. Procédé selon la revendication 1, qui comprend en outre l'étape qui consiste à combiner le matériau de détection de l'étape (b) et une certaine quantité de solution basique diluée avec le métal au moins partiellement libéré du composé organométallique.

9. Procédé selon la revendication 8, qui comprend en outre l'étape qui consiste à combiner le métal rendu basique avec un acide dilué pour oxyder le métal à un état d'oxydation qui lui permet de réagir avec la substance chimique de sensibilisation colorimétrique.

10. Procédé selon la revendication 1, dans lequel la concentration du composé organométallique dans la matrice d'hydrocarbure est déterminée en traduisant l'intensité de la couleur du complexe organométallique détectée par colorimétrie en une valeur de concentration en le métal.

11. Procédé selon la revendication 1, dans lequel le métal du composé organométallique est le manganèse.

12. Procédé selon la revendication 10, dans lequel le composé organométallique est le méthyl cyclopentadiényl manganèse tricarbonyle.

13. Système de détection en vue de détecter la présence de métaux dans une matrice d'hydrocarbure, ladite détection comportant un matériau solide de détection colorimétrique qui contient au moins une substance chimique de sensibilisation colorimétrique capable de réagir avec le métal d'un composé organométallique présent dans la matrice d'hydrocarbure.

14. Système de détection selon la revendication, qui comprend en outre une source de rayonnement qui libère du composé organométallique le métal présent dans le composé.

15. Système de détection selon la revendication 13, qui comprend en outre un moyen de détection colorimétrique de la présence d'un métal.

16. Système de détection selon la revendication 13, qui comprend un ou plusieurs réactifs capables de combiner le métal du composé organométallique avec la ou les substances chimiques de sensibilisation colorimétrique.
